# EUROPÄISCHE PATENTSCHRIFT

(11) **EP 1 310 240 B1**
(45) Veröffentlichungstag und Bekanntmachung des Hinweises auf die Patenterteilung: **19.10.2005**
(21) Anmeldenummer: 02024569.2
(22) Anmeldetag: 04.11.2002
(51) Int. Cl.: A61K 7/46, C11B 9/00, C07C 45/67, C07C 49/67

(54) **6-Alkylindan-1-one als Riechstoffe**
6-Alkylindane-1-ones as perfuming agents
6-Alkylindane-1-ones comme substances odoriférantes

(30) Priorität: 12.11.2001 DE 10155554
(43) Veröffentlichungstag der Anmeldung: 14.05.2003
(73) Patentinhaber: Bell Flavors & Fragances GmbH, 04205 Leipzig (DE)
(72) Erfinder: Sonnenberg, Steffen, Dr., 37603 Holzminden (DE); Koch, Oskar, Dr., 37079 Göttingen (DE); Duchaufour, Bertrand, 75019 Paris (FR)
(74) Vertreter: Eisenführ, Speiser & Partner

(56) Entgegenhaltungen:
- US-A- 4 532 357
- CL FERRERO ET AL: "Odeur et constitutionXVIII. Sur un dérivé à odeur musquée" HELVETICA CHIMICA ACTA, VERLAG HELVETICA CHIMICA ACTA. BASEL, CH, Bd. XVII, Nr. 238, 1959, Seiten 2111-2121, XP002157201 ISSN: 0018-019X
- G. BÜCHI ET AL: "Terpenes. IV. The Acid-induced Cyclization of Dihydro-alpha-ionone" J.AM.CHEM.SOC., Bd. 78, 1965, Seiten 2622-2625, XP002230653
- H.D. HOLLIS SHOWALTER ET AL: "Synthesis of 3,4-Dihydro-1(2H)-Isoquinolinones" J. HETEROCYCLIC CHEM. , Bd. 38, Juli 2001 (2001-07) - August 2001 (2001-08), Seiten 961-964, XP002230654
- DATABASE CROSSFIRE BEILSTEIN [Online] Beilstein Institut zur Förderung der Chemischen Wissenschaften, Frankfurt am Main, DE; Database accession no. 4405283 XP002230655 & BROUWER, D.M. ET AL.: RECL. TRAV. CHIM. PAYS-BAS, Bd. 93, 1974, Seiten 189-194,
- DATABASE CROSSFIRE BEILSTEIN [Online] Beilstein Institut zur Förderung der Chemischen Wissenschaften, Frankfurt am Main, DE; Database accession no. 3354103 XP002230656 & SASAKI, K. ET AL.: CHEM. LETT., 1988, Seiten 685-688,

## Beschreibung

Die Erfindung betrifft die Verwendung von 6-Alkylindan-1-onen als Riechstoffe, Riechstoffmischungen enthaltend 6-Alkylindan-1-one sowie parfümierte Produkte enthaltend 6-Alkylindan-1-one.

Durch die im allgemeinen nicht ausreichende Verfügbarkeit natürlicher Riech- und Aromastoffe, die sich ständig erweiternden und verändernden parfümistischen Ansprüche und der Wegfall gängiger Riechstoffe aufgrund von toxikologischen, ökologischen und ökonomischen Bedenken, besteht weiterhin der Bedarf an Verbindungen mit wertvollen Riechstoffeigenschaften. Dieser Bedarf besteht insbesondere, wenn die Riechstoffe zusätzliche positive Sekundäreigenschaften aufweisen, wie z.B. höhere Stabilität, höhere Ausgiebigkeit oder besseres Haftungsvermögen. Besonders interessant sind solche Stoffe dann, wenn sie nicht nur herausragende sensorische Eigenschaften haben, sondern wenn sich mit ihnen auch wegen ihrer Stärke und Ausgiebigkeit bemerkenswerte Effekte bei geringen Dosierungen erzielen lassen.

Riechstoffe mit einem lederartigen und holzigen Geruch sind für die parfümistische Kreation zunehmend von Interesse. Die bekannten Riechstoffe mit Ledergeruch gehören meist zu den chemischen Gruppen der Phenole oder der Chinoline. Beide chemischen Gruppen können im allgemeinen toxikologische Effekte wie Hautreizung hervorrufen.

Es besteht daher Bedarf an neuen Leder-Riechstoffen, die nicht zur chemischen Gruppe der Phenole oder Chinoline gehören und besondere sensorische Eigenschaften aufweisen.

Gegenstand der vorliegenden Erfindung ist die Verwendung von Verbindungen der Formel (**1**) 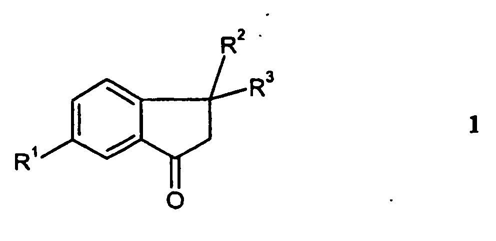 als Riechstoffe, wobei
- R¹: Methyl, Ethyl, n-Propyl, iso-Propyl, n-Butyl, iso-Butyl,
- R², R³: - unabhängig voneinander - H, Methyl, Ethyl, n-Propyl, iso-Propyl, n-Butyl, sec-Butyl, iso-Butyl
bedeuten, mit der Maßgabe, dass R² und R³ zusammen maximal 4 Kohlenstoffatome und R¹, R² und R³ zusammen maximal 8 Kohlenstoffatome enthalten.

Bevorzugt haben die Reste R¹, R² und R³ folgende Bedeutung:
- R¹: Methyl, Ethyl, iso-Propyl
- R², R³: - unabhängig voneinander - Methyl, Ethyl.

Besonders bevorzugt ist 3,3,6-Trimethylindan-1-on (R¹, R² und R³ jeweils Methyl).

Es wurde gefunden, dass die erfindungsgemäßen 6-Alkylindan-1-one einen lederartigen Geruch mit holzigen Noten aufweisen. Die erfindungsgemäßen Verbindungen zeichnen sich durch einen intensiven Geruch, einen besonders hohe Ausgiebigkeit, eine hohe geruchliche Haftung und einen wertvollen Geruchscharakter aus.

In der Patenschrift US-A 4,532,357 sind 3,3,7- und 3,3,5-Trimethylindan-1-on als Riechstoffe beschrieben. Hierin werden die geruchlichen Eigenschaften des Gemisches der beiden Isomere sowie die beiden einzelnen Isomere beschrieben.

Die sensorischen Eigenschaften der erfindungsgemäßen Verbindungen wurden in Bezug auf den Geruchscharakter, die Ausgiebigkeit, die Intensität und die Haftung im Vergleich zu den anderen Regioisomeren untersucht (siehe Tabelle 1). Alle geruchlichen Prüfungen wurden durch eine geschulte Expertengruppe mit mindestens sechs Teilnehmern mit verdeckten und codierten Proben durchgeführt.

6-Alkylindan-1-one zeichnen sich durch eine sehr hohe geruchliche Ausgiebigkeit, eine hohe geruchliche Intensität und sehr hohe geruchliche Haftung aus. Weiterhin zeichnen sich 6-Alkylindan-1-one auch durch einen deutlichen Ledergeruch mit würzigen Noten aus und unterscheiden sich hiermit von den anderen Isomeren bei denen Tabak- und Honignoten mit blumigen Aspekten dominieren.

Zur Bestimmung der Ausgiebigkeit wurden logarithmisch verdünnte Lösungen der erfindungsgemäßen Verbindung und der anderen Isomere in Diethylphthalat (DEP) hergestellt und Riechstreifen mit diesen Lösungen gleichmäßig getaucht. Anschließend wurden diese Riechstreifen im Vergleich zu Riechstreifen abgerochen, welche mit reinem und geruchlosen Diethylphthalat getaucht sind. Dadurch wurde die letzte noch riechende Verdünnung durch die Expertengruppe ermittelt. Hierbei zeigte sich, dass das 3,3,6-Trimethylindan-1-on eine fünfzig bis fünfhundertfache höhere Ausgiebigkeit als die anderen Isomere hat (Tabelle 1). Diese Eigenschaft führt dazu, dass bereits kleinste Mengen der erfindungsgemäßen Verbindungen geruchlich wahrgenommen werden können und somit in Parfümölen und parfümierten Produkten einen wesentlichen Beitrag leisten können.

Zur Bestimmung der geruchlichen Intensität der erfindungsgemäßen Verbindungen und der 3,3,4- , 3,3,5- bzw. 3,3,7-Trimethyl-Isomeren wurden die 5 gew.-%igen ethanolischen Lösungen von Riechstreifen abgerochen. Hierbei wurde die geruchliche Intensität auf einer Skala von 1 (schwach) bis 9 (sehr stark) im Vergleich beurteilt. Hierbei zeigt sich, dass beispielsweise das 3,3,6-Trimethylindan-1-on eine deutlich höhere geruchliche Intensität hat als die anderen Isomere (Tabelle 1).

Die geruchliche Haftung bezeichnet die geruchliche Wahrnehmung von Riechstoffen über einen langen Zeitraum. Zur Bewertung der geruchlichen Haftung evaluiert die Expertengruppe jeweils täglich Riechstreifen mit der erfindungsgemäßen Verbindung und den anderen Isomeren bis zu dem Zeitpunkt, an dem kein oder nur noch ein sehr schwacher bzw. untypischer Geruch wahrgenommen werden kann. Hierbei zeigte sich, dass das 3,3,6-Trimethylindan-1-on eine deutlich längere geruchliche Haftung besitzt als die anderen untersuchten isomeren Trimethylindan-1-one (Tabelle 1).

**Tabelle 1**

| Geruchliche Eigenschaften von Trimethylindan-1-onen | | | | |
|---|---|---|---|---|
| Substanz | Geruchscharakter | Ausgiebigkeit [10⁻⁶% in DEP] | Intensität | Haftung [Tage] |
| 3,3,4-Trimethylindanon | Wachsig, nussig, würzig, ledrig, Labdanum | 500 | 6 | 8 |
| 3,3,5-Trimethylindanon | Grüne Pepperoni, Isobutylchinolin, ledrig, Spargel, grüne Bohnen | 100 | 5 | 9 |
| 3,3,6-Trimethylindanon | Ledrig, Labdanum, balsamisch, würzig, fruchtig | 1 | 7 | 12 |
| 3,3,7-Trimethylindanon | Honigartig, holzig, Bienenwachs, leicht rauchig | 50 | 6 | 8 |

Aufgrund der durchgeführten sensorischen Arbeiten wurde überraschenderweise gefunden, dass das 3,3,6-Trimethylindan-1-on wesentliche Vorteile gegenüber den anderen Isomeren aufweist.

Für die Beurteilung der einzelnen isomeren Trialkylindan-1-one wurden diese durch Umsetzung von alkylsubstituierten Benzoesäure-isobutylestern in Polyphosphorsäure hergestellt. So konnten beispielsweise die 3,3,4-, 3,3,5-, 3,3,6- und 3,3,7-Trimethylindan-1-one durch Umsetzung von *para-*, *meta-* bzw. *ortho*-Tolylsäure-isobutylester in Polyphosphorsäure erhalten werden.

Auch im Gemisch mit anderen isomeren Trialkylindan-1-onen wie z.B. 3,3,4-, 3,3,5- oder 3,3,7-Trimethylindan-1-on entfalten die erfindungsgemäßen Verbindungen einen angenehmen lederartigen Geruch, der in Parfümkompositionen einen wertvollen Beitrag leistet. Überraschenderweise können die erfindungsgemäßen Verbindungen in einem Gemisch mit isomeren Trimethylindan-1-onen einen besonders intensiven Geruch verleihen und eine besonders hohe Ausgiebigkeit erzeugen, der durch die beiden Isomere allein nicht zu erzielen ist. Hierdurch ergeben sich erhebliche Vorteile bei der Anwendung der 6-Alkylindan-1-one bei der Verwendung in Parfümölen.

Die erfindungsgemäßen Verbindungen können sowohl als einzelner Riechstoff, als auch im Gemisch mit isomeren Trimethylindan-1-onen bzw. zusammen mit anderen Riechstoffen überraschende verstärkende Effekte in Parfümölen erzeugen.

Beispiele für Riechstoffe in den Parfümölen finden sich z.B. in K. Bauer, D. Garbe und H. Surburg, Common Fragrance and Flavor Materials, 3^{rd}. Ed., Wiley-VCH, Weinheim 1997.

Im einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklävendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexannoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpineol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,-6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha-3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexenearbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-.tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die Herstellung der erfindungsgemäßen Verbindungen ist für einige Verbindungen beschrieben. In Journal of Am.Chem. Soc. 78, 2622 (1956) ist beispielsweise die Herstellung von 3,3,6-Trimethylindan-1-on beschrieben. Diese dreistufige Synthese ist insgesamt unbefriedigend, sowohl in Bezug auf die Reaktionsbedingungen als auch auf die Ausbeute (40% der Theorie über drei Stufen).

Es wurde nun gefunden, dass die Herstellung von 1-Indanonen durch Umsetzung von (3,3-alkyl-substituierter) Acrylsäure (**3**) oder deren Ester in einer Friedel-Crafts-Reaktion mit (Alkyl-)benzolen (**2**) und anschließende Cyclisierung des gebildeten Zwischenproduktes (**4**) in hohen Ausbeuten möglich ist, wobei für diese Cyclisierung Polyphosphorsäure der bevorzugte Katalysator ist.

Das Herstellverfahren kann durch folgendes Reaktionsschema erläutert werden:

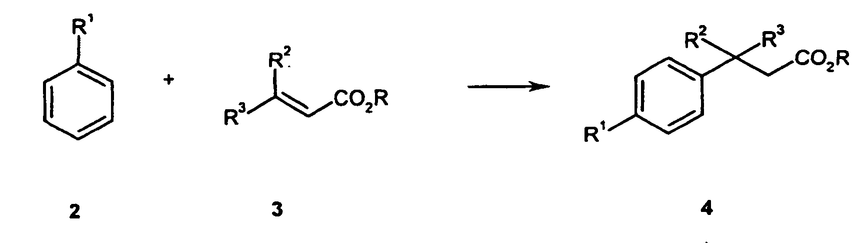

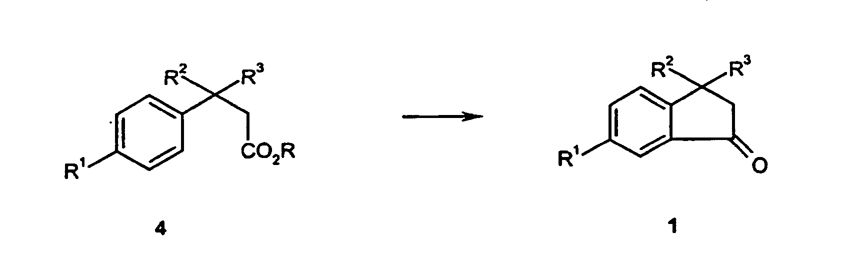

Die Reste R, R¹, R² und R³ können dabei folgende Bedeutung haben:
- R: Wasserstoff, C₁ - C₄ Alkyl,
- R¹: Wasserstoff, Methyl, Ethyl, n-Propyl, iso-Propyl, n-Butyl, iso-Butyl,
- R², R³: unabhängig voneinander - H, Methyl, Ethyl, n-Propyl, iso-Propyl, n-Butyl, sec-Butyl, iso-Butyl.

Bevorzugt haben die Reste R, R¹, R² und R³ folgende Bedeutung:
- R: Wasserstoff, Methyl, Ethyl,
- R¹: Methyl, Ethyl, iso-Propyl
- R², R³: - unabhängig voneinander - Methyl, Ethyl.

Besonders bevorzugt ist R = H und R¹, R² und R³ jeweils Methyl.

In der 1. Stufe, d.h. der Umsetzung der (Alkyl)-benzole (**2**) mit dem AcrylsäureDerivat (**3**), werden bevorzugt Friedel-Crafts-Katalysatoren verwendet. Als Friedel-Crafts-Katalysatoren können alle für diesen Reaktionstyp üblichen Katalysatoren verwendet werden. Besonders geeignet sind Metallhalogenide wie beispielsweise Aluminiumchlorid, Eisenchlorid oder Zinkchlorid. Besonders bevorzugt ist Aluminiumchlorid. Die Friedel-Crafts-Reaktion kann in einem breiten TemperaturBereich durchgeführt werden. Die Friedel-Crafts-Reaktion wird vorteilhafterweise bei Temperaturen im Bereich von -20 bis 100°C, bevorzugt im Bereich von -10 bis 50°C und besonders bevorzugt im Bereich von 0 bis 25°C durchgeführt.

Die 1. Stufe kann in Gegenwart von nicht aromatischen Lösungsmitteln durchgeführt werden, wie z.B. Ether (z.B. Methyl-tert.butylether, Tetrahydrofuran), cyclische oder acyclische Kohlenwasserstoffe (z.B. Cyclohexan). Die 1. Stufe wird bevorzugt lösungsmittelfrei durchgeführt. Bevorzugt ist ein Überschuss an (Alkyl-)benzol (**2**).

Die erhaltenen Zwischenprodukte (**4**) sind von ausreichender Reinheit, um zum Endprodukt (**1**) umgesetzt werden zu können. Bei Bedarf kann eine Reinigung erfolgen, beispielsweise mittels Destillation.

Die 2. Stufe, d.h. die Cyclisierung des Zwischenproduktes (**4**) zu den erfindungsgemäßen 6-Alkylindan-1-onen (**1**), erfolgt bevorzugt in Gegenwart einer Säure und einem wasserbindenden Mittel. Besonders bevorzugter Katalysator für die 2. Stufe ist Polyphosphorsäure. Bevorzugte Mengen sind 50 bis 250 Gew.-% bezogen auf eingesetztes Substrat, bevorzugte Gehalte der Polyphophorsäure liegen bei 110-120 % bezogen auf Moläquivalente Phosphorsäure. Die Cyclisierung kann vorteilhafterweise bei Temperaturen im Bereich von 50 bis 180°C, bevorzugt im Bereich von 80 bis 150°C und besonders bevorzugt im Bereich von 100 bis 130°C durchgeführt werden.

### Beispiele

### Beispiel 1 3,3,6-Trimethyl-indan-1-on

### a) 3-Methyl-3-(4'-methylphenyl)-buttersäure

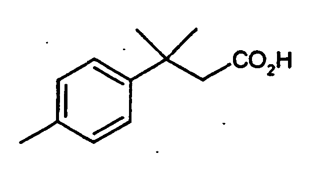

In 300 g (3,26 mol) Toluol wurden 340 g (2,6 mol) Aluminiumchlorid bei Raumtemperatur eingerührt und auf 0°C abgekühlt. Danach wurde bei 0-5°C eine Lösung von 250 g (2,5 mol) 3,3-Dimethylacrylsäure in 400 g (4,34 mol) Toluol innerhalb 1h zudosiert. Nach Dosierende wurde noch 10 min nachgerührt, das Reaktionsgemisch auf 1000 g verdünnte Salzsäure gegeben und mit Methyl-tert.-butylether extrahiert. Nach Destillation lagen 370 g Produkt vor mit einer GC-Reinheit von 95 %. Ausbeute 77 % d. Theorie

### b) 3,3,6-Trimethyl-indan-1-on

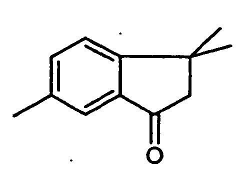

730 g Polyphoshorsäure (115 % bezogen auf Moläquivalente Phosphorsäure) wurden vorgelegt und auf 120°C aufgeheizt. Danach wurden 370 g (1,93 mol) Tolylbuttersäure (Rohprodukt aus der 1. Stufe, Beispiel 1a) während 45 min. zudosiert und noch 3 h bei 120°C nachgerührt. Es wurde auf 60°C abgekühlt und mit 900 g Eiswasser versetzt, mit Methyl-tert.-butylether extrahiert und anschließend destilliert. Es wurden 320 g Produkt 3,3,6-Tri-methylindan-1-on mit einer GC-Reinheit von 95 % erhalten. Ausbeute 95 % d. Theorie.

### Anwendunsgtechnische Beispiele

### Beispiel 2 Parfümkomposition 1 und 2

| Name | Gewichtsteile Komposition 1 | Gewichtsteile Komposition 2 |
|---|---|---|
| Red Berries Extrakt | 40 | 40 |
| Bergamottöl bergaptenfrei | 80 | 80 |
| Cardamomen absolue | 25 | 25 |
| Cyclopentadecanolid (1) | 190 | 190 |
| Cypressenöl | 5 | 5 |
| Eugenol | 50 | 50 |
| Ingweröl synthetisch | 60 | 60 |
| Methyldihydrojasmonat | 100 | 100 |
| Iso E Super® (2) | 200 | 200 |
| Amarocit® (1) | 50 | 50 |
| Muscon | 50 | 50 |
| Muskateller Salbeiöl | 80 | 80 |
| Stemone®(3) | 10 | 10 |
| Vertocitral® (1) | 50 | 50 |
| Vetiveröl | 10 | 10 |
| 3,3,6-Trimethyl-indan-1-on | 1 | - |

| | | |
|---|---|---|
| (1) Haarmann & Reimer, Deutschland, Holzminden | | |
| (2) International Flavors & Fragrances, USA, New Jersey, Union Beach | | |
| (3) Givaudan, Schweiz, Genf | | |

Der Zusatz von 3,3,6-Trimethylindan-1-on zu Parfümkomposition 1 bewirkt eine deutliche Intensivierung des Gesamtgeruches, wobei zusätzlich noch die ledrigen Noten verstärkt werden. Weiterhin bekommt die Parfümkomposition einen deutlich herberen Charakter, der für einen Herrenduft besonders geeignet ist.

### Beispiel 3 Parfümkomposition 3 und 4

| Name | Gewichtsteile Komposition 3 | Gewichtsteile Komposition 4 |
|---|---|---|
| Ambrarome abs.® (4) | 2 | 2 |
| Benzoe Resin | 100 | 100 |
| Bergamottöl bergaptenfrei | 12 | 12 |
| Citral | 5 | 5 |
| Zibeth Tinktur 10% in Dipropylenglycol | 20 | 20 |
| Coumarin | 19 | 19 |
| Indol 10% in Dipropylenglycol | 5 | 5 |
| Isomethylionon | 30 | 30 |
| Linalool | 10 | 10 |
| Opoponaxöl | 5 | 5 |
| Patchoulyöl | 20 | 20 |
| Tolubalsam Resin farblos | 2 | 2 |
| Vanille Resin entfärbt 10% in Dipropylenglycol | 50 | 50 |
| 3,3,6-Trimethyl-indan-1-on | 5 | - |

| | | |
|---|---|---|
| (4) Synarome, Frankreich, Bois Colombes | | |

Durch den Zusatz von 3,3,6-Trimethyl-indan-1-on zu Parfümkomposition 3 wird bei dieser orientalisch blumigen Parfümkomposition eine Harmonisierung des gesamten Geruches erreicht. Die Parfümkomposition riecht natürlicher und wurde in einem Präferenztest eindeutig gegenüber Parfümkomposition 4 bevorzugt.

### Beispiel 4 Parfümkomposition 5 und 6

| Name | Gewichtsteile Komposition 5 | Gewichtsteile Komposition 6 |
|---|---|---|
| Muskateller Salbeiöl synth. | 100 | 100 |
| Davanaöl | 10 | 10 |
| Benzylsalicylat | 100 | 100 |
| Undecavertol® (3) | 5 | 5 |
| Irisöl synth. | 5 | 5 |
| Irone | 2 | 2 |
| Coumarin | 80 | 80 |
| Nectarol® (5) | 2 | 2 |
| Zedernholzöl Florida | 100 | 100 |
| Andrane® (2) | 100 | 100 |
| Sclareolide | 10 | 10 |
| Ambroxid (1) | 5 | 5 |
| Isomerengemisch aus gleichen Anteilen von 3,3,4-, 3,3,5-, 3,3,6- und 3,3,7-Trimethyl-indan-1-on | 200 | - |

| | | |
|---|---|---|
| (1) Haarmann & Reimer, Deutschland, Holzminden | | |
| (2) International Flavors & Fragrances, USA, New Jersey, Union Beach | | |
| (3) Givaudan, Schweiz, Genf | | |
| (5) Firmenich, Schweiz, Genf | | |

Die Parfümkomposition 5 hat einen signifikant intensiveren Geruch, bei dem die ledrige Labdanumnote sich harmonisch in das Geruchsbild einbringt. Bei der insgesamt geruchlich schwächeren Parfümkomposition 6 ist die ledrige Note deutlich geringer. Die Parfümkomposition ist deutlich besser geeignet für die Parfümierung einer Pflegeserie für Herren.

### Beispiel 5 Shampoo

Die Parfümkompositionen 1 und 2 wurden separat zu jeweils 1 Gew.-% in die unten aufgeführte Shampooformulierung (Tabelle 2) eingearbeitet und das Shampoo zum Waschen von Haar verwendet. Anschließend erfolgte die geruchliche Evaluierung durch eine Expertengruppe aus der Shampoomasse, der verdünnten wässerigen Lösung im Haar, dem nassen ausgespülten Haar und dem getrockneten Haar.

Der Zusatz von 3,3,6-Trimethyl-indan-1-on verleiht der Parfümkomposition 1 im Vergleich zur Parfümkomposition 2 in allen Evaluierungsschritten des Shampoos eine hervorragende abgerundete Ledernote. Besonders auf dem nassen und dem trockenen Haar ist der ledrige Geruch deutlich wahrnehmbar und stellt eindeutig eine Verbesserung der Parfümkomposition dar.

**Tabelle 2**

| Shampooformulierung | | |
|---|---|---|
| Inhaltsstoffe | INCI-Bezeichnung | % (w/w) |
| Entmineralisiertes Wasser | Water (Aqua) | 75,45 |
| Plantacare PS 10 (1) | Sodium Laureth Sulfate (and) Lauryl Glycoside | 20,00 |
| Natriumchlorid | Sodium Chloride | 1,40 |
| Citronensäure 10,0 % Lösung | Citric Acid | 1,65 |
| Phenonip (2) | Phenoxyethanol (and) Methyl paraben (and) Ethylparaben (and) Propylparaben (and) Butylparaben | 0,50 |
| Komposition 1 bzw. 2 (3) | Fragrance | 1,00 |

### Lieferanten:

(1) Cognis Deutschland GmbH, D-40191 Düsseldorf, Germany
(2) Nipa Laboratories Ltd., CF382SN South Wales, UK
(3) Haarmann & Reimer, D-37603 Holzminden, Germany

### Beispiel 6 Waschpulver

Die Parfümkompositionen 5 und 6 wurden separat zu jeweils 0,4 Gew.-% in eine Waschmittelformulierung (Tabelle 3) eingearbeitet. Anschließend wurde der Geruch aus dem Pulver, von der wässerigen Lösung, der feuchten und der trockenen Wäsche durch eine Expertengruppe untersucht. Es wurde gefunden, dass die Parfümkomposition 5 allgemein eine höhere Präferenz hatte und durch die holzigen und ledrigen Noten einen harmonischere Duft erzeugte. Insbesondere auf der feuchten und der trocknen Wäsche wurde von der Parfümkomposition 5 im Vergleich zur Komposition 6 ein deutlich intensiverer und typischer ledriger Geruch wahrgenommen.

**Tabelle 3**

| Waschpulverformulierung | | |
|---|---|---|
| Inhaltsstoffe | INCI-Bezeichnung | % (w/w) |
| Marlon A (1) | Sodium laurylbenzene sulphonate, approx. 80% | 7,0 % |
| Marlipal 013 (1) | C13/15-Oxoalcoholpolyglycolether | 4,0 % |
| Prisavon (2) | Soybean/Coco soap, approx. 25 % fatty acid | 3,5 % |
| Thylose (3) | Carboxymethylcellulose | 1,5 % |
| Tinopal (4) | Optical brightener | 0,2 % |
| Optimase (5) | Enzymes | 0,6 % |
| Wasserglas (6) | Sodium disilicate | 5,0 % |
| Natriumsulfat (6) | Sodium sulphate (anhydrous) | 26,8 % |
| Natriumperborat (1) | Sodium perborate (tetrahydrate) | 20,0 % |
| Natriumtripolyphosphat (1) | Sodium tripolyphosphate or Zeolithe | 26,0 % |
| Magnesiumsilikat (6) | Magnesium silicate | 2,0 % |
| TAED (3) | Bleach activator | 3,0 % |
| Komposition 5 bzw. 6 (7) | Fragrance | 0,4 % |

### Lieferanten:

(1) Degussa-Hüls AG, D-60287 Frankfurt, Germany
(2) Uniqema Chemie, NL-2800 AA Gouda, Netherlands
(3) Clariant GmbH, D-29699 Frankfurt, Germany
(4) Ciba Spezialitätenchemie AG, 4000 Basel, Switzerland
(5) Genecore Int. GmbH, D-31564 Nienburg, Germany
(6) Merck KGaA, D-64293 Darmstadt, Germany
(7) Haarmann & Reimer GmbH, D-37603 Holzminden, Germany

## Patentansprüche

1. Verwendung von Verbindungen der Formel (**1**) 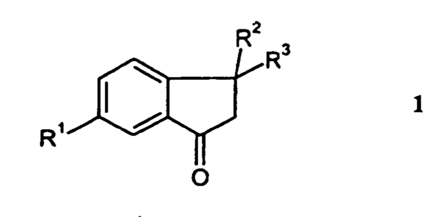 als Riechstoffe, wobei
R¹ Methyl, Ethyl, n-Propyl, iso-Propyl, n-Butyl, iso-Butyl,
R², R³ - unabhängig voneinander - H, Methyl, Ethyl, n-Propyl, iso-Propyl, n-Butyl, sec-Butyl, iso-Butyl
bedeuten, mit der Maßgabe, dass R² und R³ zusammen maximal 4 Kohlenstoffatome und R¹, R² und R³ zusammen maximal 8 Kohlenstoffatome enthalten.

2. Parfümierte Produkte, enthaltend mindestens eine Verbindung der Formel (**1**) aus Anspruch 1.

3. Riechstoffmischungen, enthaltend mindestens eine Verbindung der Formel (**1**) aus Anspruch 1.

## Claims

1. Use of compounds of formula (1) 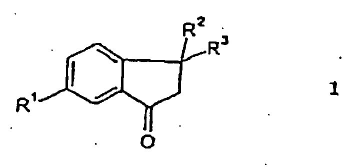 as fragrances, wherein
R¹ represents methyl, ethyl, n-propyl, isopropyl, n-butyl, isobutyl,
R², R³ - independently of one another - represent H, methyl, ethyl, n-propyl, isopropyl, n-butyl, sec.-butyl, isobutyl,
with the proviso that R² and R³ together contain not more than 4 carbon atoms and R¹, R² and R³ together contain not more than 8 carbon atoms.

2. Perfumed products comprising at least one compound of formula (1) from claim 1.

3. Fragrance mixtures comprising at least one compound of formula (1) from claim 1.

## Revendications

1. Utilisation de composés de formule (1) 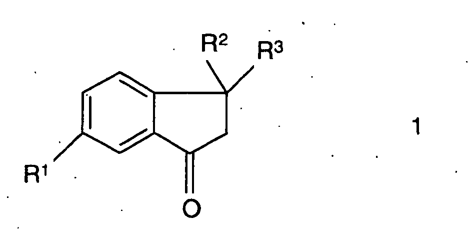 comme substances odorantes où
R¹ représente méthyle, éthyle, n-propyle, isopropylé, n-butyle, isobutyle,
R², R³ - indépendamment l'un de l'autre - représentent H, méthyle, éthyle, n-propyle, isopropyle, n-butyle, sec-butyle, isobutyle,
avec la condition que R² et R³ contiennent ensemble au maximum 4 atomes de carbone et que R¹, R² et R³ contiennent ensemble au maximum 8 atomes de carbone.

2. Produits parfumés contenant au moins un composé de formule (1) selon la revendication 1.

3. Mélanges de substances odorantes contenant au moins un composé de formule (1) selon la revendication 1.
